# EUROPEAN PATENT SPECIFICATION

(11) **EP 3 820 380 B1**
(45) Date of publication and mention of the grant of the patent: **22.04.2026**
(21) Application number: 19745522.3
(22) Date of filing: 10.07.2019
(51) Int. Cl.: A61B 17/12, A61M 25/10

(54) **OCCLUSIVE DEVICE WITH EXPANDABLE MEMBER**
VERSCHLUSSVORRICHTUNG MIT EXPANDIERBAREM ELEMENT
DISPOSITIF OCCLUSIF AVEC ÉLÉMENT EXTENSIBLE

(30) Priority: 10.07.2018 US 201862695985 P
(43) Date of publication of application: 19.05.2021
(73) Proprietor: Boston Scientific Scimed, Inc., Maple Grove, MN 55311 (US)
(72) Inventor: ANDERSON, James M., Corcoran, Minnesota 55357 (US); ONUSHKO, David John, Maple Grove, Minnesota 55369 (US); INOUYE, Joshua Mark, Maple Grove, Minnesota 55369 (US)
(74) Representative: Peterreins Schley
(86) International application number: PCT/US2019/041138
(87) International publication number: WO 2020/014312

(56) References cited:
- WO-A1-2008/010792
- WO-A1-2014/063039
- WO-A1-2015/164836
- WO-A1-2018/017935
- WO-A2-2008/139461
- CN-A- 106 859 722
- US-A1- 2005 288 706
- US-A1- 2016 100 843

## Description

### BACKGROUND

The left atrial appendage (LAA) is a small organ attached to the left atrium of the heart as a pouch-like extension. In patients suffering from atrial fibrillation, the left atrial appendage may not properly contract with the left atrium, causing stagnant blood to pool within its interior, which can lead to the undesirable formation of thrombi within the left atrial appendage. Thrombi forming in the left atrial appendage may break loose from this area and enter the blood stream. Thrombi that migrate through the blood vessels may eventually plug a smaller vessel downstream and thereby contribute to stroke or heart attack. Clinical studies have shown that the majority of blood clots in patients with atrial fibrillation are found in the left atrial appendage. As a treatment, medical devices have been developed which are positioned in the left atrial appendage and deployed to close off the ostium of the left atrial appendage. Over time, the exposed surface(s) spanning the ostium of the left atrial appendage becomes covered with tissue (a process called endothelization), effectively removing the left atrial appendage from the circulatory system and reducing or eliminating the number of thrombi which may enter the blood stream from the left atrial appendage. A continuing need exists for improved medical devices and methods to control thrombus formation within the left atrial appendage of patients suffering from atrial fibrillation.
WO 2015/164836 A1 and WO 2018/017935 A1 relate to left atrial appendage occluison device. WO2015/164836 discloses an expandable member including a first balloon defining a first inflation chamber, a second balloon defining a second inflation chamber, with the second inflation chamber positioned adjacent to the first inflation chamber. The expandable member is designed to shift between a first unexpanded configuration and a second expanded configuration. The device further comprises a first inflation valve member extending at least partially into the first inflation chamber, wherein the expandable member is configured to expand and seal the opening of the left atrial appendage.
US 2016/100843 A1 relates to cardiovascular devices for closure of an opening or isolation of a structure in the cardiovascular system. WO 2014/063039 A1 relates to reinforced inflatable medical devices. US 2005/288706 A1 relates to an inflatable occluder. CN 106 859 722 A relates to a balloon left atrial appendage closure device.

### SUMMARY

The invention is defined by independent claim 1. Further embodiments of the invention are defined by the dependent claims.

This disclosure provides design, material, manufacturing method, and use alternatives for medical devices. An example medical device for occluding the left atrial appendage, not part of the present invention, includes an expandable member including a first balloon defining a first inflation chamber and a second balloon defining a second inflation chamber.

Further, the second inflation chamber is positioned adjacent to the first inflation chamber, the first inflation chamber is in fluid communication with the second inflation chamber and the expandable member is designed to shift between a first configuration and a second expanded configuration. Additionally, the first balloon is designed to fill a first region of the left atrial appendage and the second balloon is designed to fill a second region of the left atrial appendage. The medical device also includes a first inflation valve member extending at least partially into the first inflation chamber and the expandable member is configured to expand and seal the opening of the left atrial appendage.

Alternatively or additionally to any of the embodiments above, wherein the expandable member is configured to inflate the first chamber to a first inflation pressure, and wherein the expandable member is configured to inflate the second chamber to a second inflation pressure after the first chamber is inflated to the first inflation pressure.

Alternatively or additionally to any of the embodiments above, further comprising one or more attachment arms positioned adjacent to the expandable member, the one or more attachment arms including one or more projections disposed thereon, wherein the one or more projections are configured to engage a portion of the left atrial appendage wall.

Alternatively or additionally to any of the embodiments above, wherein the expandable member includes a first one-way valve configured to permit an inflation material to flow between the first chamber and the second chamber, wherein the first one-way valve is configured to open when the first chamber is inflated to a first threshold inflation pressure.

Alternatively or additionally to any of the embodiments above, wherein the expandable member further comprises a third inflation chamber in fluid communication with the first inflation chamber, a fourth inflation chamber in fluid communication with the first inflation chamber, and a fifth inflation chamber in fluid communication with the first inflation chamber.

Alternatively or additionally to any of the embodiments above, wherein the second inflation chamber, the third inflation chamber, the fourth inflation chamber and the fifth inflation chamber are spaced circumferentially around the first inflation chamber.

Alternatively or additionally to any of the embodiments above, further comprising a projection configured to anchor the medical device to a target tissue site of the left atrial appendage.

Alternatively or additionally to any of the embodiments above, wherein the projection is configured to shift between a first position and a second extended position, wherein the projection extends radially away from an outer surface of the expandable member in the second extended position.

Alternatively or additionally to any of the embodiments above, wherein the first inflation chamber is positioned proximal to the second inflation chamber.

Alternatively or additionally to any of the embodiments above, further comprising a second inflation valve positioned between the first inflation chamber and the second inflation chamber.

Alternatively or additionally to any of the embodiments above, wherein the expandable member is designed to engage an inflation catheter having a first inflation port and a second inflation port, and wherein the first inflation port extends into the second inflation chamber through the second inflation valve, and wherein the second inflation port is positioned within the first inflation chamber when the first inflation port is positioned within the second inflation chamber.

Alternatively or additionally to any of the embodiments above, wherein the expandable member is configured to inflate the second chamber to a first inflation pressure, and wherein the expandable member is configured to inflate the first chamber to a second inflation pressure after the second chamber is inflated to the first inflation pressure, and wherein the second inflation valve is designed to maintain the first inflation pressure in the second chamber while the first inflation chamber is inflated to the second inflation pressure.

Another medical device not part of the present invention for occluding the left atrial appendage includes:
an expandable balloon including an outer surface, a first lobe defining a first inner expansion cavity and a second lobe defining a second inner expansion cavity positioned adjacent to the first inner expansion cavity, wherein the first lobe is designed to expand into a first region of the left atrial appendage and the second lobe is designed to expand into a second region of the left atrial appendage;
a first valve member positioned between the first inner expansion cavity and the second inner expansion cavity, wherein the first valve member is designed to seal the first inner expansion cavity from the second inner expansion cavity;
wherein the expandable balloon is configured to expand and seal the opening of the left atrial appendage.

Alternatively or additionally to any of the embodiments above, wherein the first valve member permits fluid communication between the first inner expansion cavity and the second inner expansion cavity.

Alternatively or additionally to any of the embodiments above, wherein the first chamber is designed to inflate to a first inflation pressure, and wherein the second chamber is designed to inflate to a second inflation pressure after the first chamber is inflated to the first inflation pressure.

Alternatively or additionally to any of the embodiments above, wherein the valve is configured to open when the first chamber is inflated to a first threshold inflation pressure.

Alternatively or additionally to any of the embodiments above, further comprising a second valve member positioned proximal to the first valve member.

Alternatively or additionally to any of the embodiments above, wherein both the first valve member and the second valve member are configured to permit an inflation catheter to extend therethrough.

An example method for sealing the left atrial appendage, not part of the present invention, includes:
advancing an expandable occluder to a position adjacent the left atrial appendage, wherein the expandable occluder includes:
   an expandable member having a first lobe defining a first inflation chamber and a second lobe defining a second inflation chamber, the second inflation chamber positioned adjacent to the first inflation chamber, wherein the first inflation chamber is in fluid communication with the second inflation chamber; and
   a first inflation valve member extending at least partially into the first inflation chamber;
inserting a tubular member into the valve;
passing an inflation media through the tubular member into the valve; and
inflating the expandable member to a first position such that the first lobe is positioned within a first region of the left atrial appendage;
inflating the expandable member to a first position such that the second lobe is positioned within a second region of the left atrial appendage.

Alternatively or additionally to any of the embodiments above, further comprising:
inflating the expandable member to a second position in which the first lobe of the expandable member seals against an inner surface of the first region of the left atrial appendage and the second lobe of the expandable member seals against an inner surface of the second region of the left atrial appendage.

The above summary of some embodiments is not intended to describe each disclosed embodiment or every implementation of the present disclosure. The Figures, and Detailed Description, which follow, more particularly exemplify these embodiments

### BRIEF DESCRIPTION OF THE DRAWINGS

The disclosure may be more completely understood in consideration of the following detailed description of various embodiments in connection with the accompanying drawings, in which:
FIG. 1 is a perspective view of an example occlusive implant;
FIG. 2 shows a cross-sectional view along line 2-2 of FIG. 1;
FIG. 3 shows a cross-sectional view along line 3-3 of FIG. 1;
FIG. 4 is an end view of the example occlusive implant shown in FIG. 1 in an unexpanded configuration;
FIG. 5 is an end view of the example occlusive implant shown in FIG. 1 in an expanded configuration;
FIG. 6 illustrates an example occlusive implant positioned in an opening of the left atrial appendage;
FIGS. 7-9 illustrate an example occlusive implant being inflated within an opening of the left atrial appendage;
FIG. 10 illustrates a cross-sectional view of another example occlusive implant;
FIG. 11 illustrates another example occlusive implant;
FIG. 12 illustrates a partial cross-sectional view of the occlusive implant show in FIG. 11;
FIG. 13 illustrates an inflation catheter positioned within the occlusive implant shown in FIG. 12;
FIG. 14 illustrates another occlusive implant;
FIG. 15 illustrates the occlusive implant shown in FIG. 14 positioned in the left atrial appendage;
FIG. 16 illustrates another occlusive implant;
FIG. 17 illustrates the occlusive implant shown in FIG. 16 positioned in the left atrial appendage;
FIG. 18 illustrates another occlusive implant;
FIG. 19 illustrates the occlusive implant shown in FIG. 18 positioned in the left atrial appendage.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the disclosure.

### DETAILED DESCRIPTION

The following description should be read with reference to the drawings, which are not necessarily to scale, wherein like reference numerals indicate like elements throughout the several views. The detailed description and drawings are intended to illustrate but not limit the claimed disclosure. Those skilled in the art will recognize that the various elements described and/or shown may be arranged in various combinations and configurations without departing from the scope of the disclosure. The detailed description and drawings illustrate example embodiments of the claimed disclosure. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). **In** many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete elements together.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The occurrence of thrombi in the left atrial appendage (LAA) during atrial fibrillation may be due to stagnancy of blood pooling in the LAA. The pooled blood may still be pulled out of the left atrium by the left ventricle, however less effectively due to the irregular contraction of the left atrium caused by atrial fibrillation. Therefore, instead of an active support of the blood flow by a contracting left atrium and left atrial appendage, filling of the left ventricle may depend primarily or solely on the suction effect created by the left ventricle. However, the contraction of the left atrial appendage may not be in sync with the cycle of the left ventricle. For example, contraction of the left atrial appendage may be out of phase up to 180 degrees with the left ventricle, which may create significant resistance to the desired flow of blood. Further still, most left atrial appendage geometries are complex and highly variable, with large irregular surface areas and a narrow ostium or opening compared to the depth of the left atrial appendage. For example, in some instances the left atrial appendage may include a bifurcated (e.g., multi-lobe) shape. These aspects as well as others, taken individually or in various combinations, may lead to high flow resistance of blood out of the left atrial appendage.

In an effort to reduce the occurrence of thrombi formation within the left atrial appendage and prevent thrombi from entering the blood stream from within the left atrial appendage, it may be desirable to develop medical devices and/or occlusive implants that close off the left atrial appendage from the heart and/or circulatory system, thereby lowering the risk of stroke due to thromboembolic material entering the blood stream from the left atrial appendage. For example, in some instances it may be desirable to develop medical devices which are designed to close off a bifurcated (e.g., multi-lobe) left atrial appendage. Example medical devices and/or occlusive implants that close off the left atrial appendage are disclosed herein.

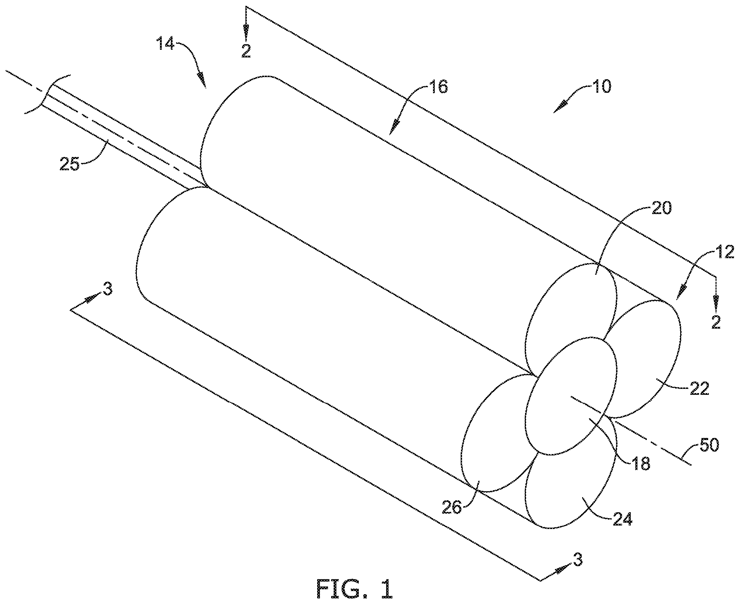

FIG. 1 illustrates an example occlusive implant 10. The occlusive implant 10 may include a first end region 12 and a second end region 14. As will be discussed in greater detail below, the first end region 12 may include the portion of the occlusive implant 10 which extends farthest into a left atrial appendage, while the second end region 14 may include the portion of the occlusive implant 10 which is positioned closer to an opening of the left atrial appendage.

The occlusive implant 10 may include an expandable member 16. The expandable member 16 may also be referred to as an expandable balloon 16. The expandable member 16 may be formed from a highly compliant material (e.g., "inflation material") which permits the expandable member 16 to expand from a first unexpanded (e.g., deflated, collapsed, delivery) configuration to a second expanded (e.g., inflated, delivered) configuration. In some examples, one or more portions of the expandable balloon 16 may be inflated to pressures from about 6895 Pa to 1379000 Pa (1 psi to about 200 psi). It can be appreciated that the outer diameter of the occlusive implant 10 may be larger in the expanded configuration versus the unexpanded configuration. Example materials used for the inflation material may be hydrogel beads (or other semi-solid materials), saline, etc.

In some examples, the expandable member 16 may be constructed from silicone or a low-durometer polymer, however, other materials are contemplated. Additionally, the expandable member 16 may be impermeable to blood and/or other fluids, such as water. In some embodiments, the expandable member 16 may include a woven, braided and/or knitted material, a fiber, a sheet-like material, a metallic or polymeric mesh, or other suitable construction. Further, in some embodiments, the expandable member 16 may prevent thrombi (e.g., blood clots, etc.) originating in the left atrial appendage from passing through the occlusive device 10 and into the blood stream. In some embodiments, the occlusive device 10 may promote endothelial growth after implantation, thereby effectively removing the left atrial appendage from the patient's circulatory system. Some suitable, but non-limiting, examples of materials for the occlusive member 10 are discussed below.

FIG. 1 further illustrates that expandable member 16 may include multiple balloons spaced circumferentially around a central balloon member. For example, FIG. 1 illustrates that the expandable member 16 may include a first central balloon member 18 extending along the longitudinal axis 50 of the expandable member 16 from the second end region 14 to the first end region 12. Additionally, FIG. 1 illustrates that the expandable member 16 may include a second expandable balloon 20, a third expandable balloon 22, a fourth expandable balloon 24 and a fifth expandable balloon 26 circumferentially spaced around the first expandable balloon member 18.

It can be appreciated from FIG. 1 that positioning of adjacent expandable balloons may be substantially uniform around the circumference of the first expandable balloon 18. Additionally, it can be appreciated that while the expandable member 16 shown in FIG. 1 includes four balloons spaced around the first central balloon member 18, this is not intended to be limiting. Rather, it can be appreciated that more or less than four balloons may be spaced around the first central balloon member 18. For example, the expandable member 16 may include 1, 2, 3, 4, 5, 6 or more distinct balloon members spaced around the first central balloon member 18. According to the claimed invention the expandable member comprises at least two distinct balloon members spaced around the first central balloon member.

Additionally, in some examples each of the second expandable balloon 20, the third expandable balloon 22, the fourth expandable balloon 24 and/or the fifth expandable balloon 26 may be formed as a monolithic structure with the first expandable balloon member 18. In other words, portions of the various balloon members may be shared among the balloon members (e.g., adjacent balloon members may share a balloon wall). However, in other examples one or more of the second expandable balloon 20, the third expandable balloon 22, the fourth expandable balloon 24 and/or the fifth expandable balloon 26 may be separate distinct from one another and/or formed from a different material from each other and/or the first central balloon member 18. Some suitable, but non-limiting, examples of materials for the balloon members are discussed below.

FIG. 1 illustrates that each of the first expandable balloon 18, the second expandable balloon 20, the third expandable balloon 22, the fourth expandable balloon 24 and/or the fifth expandable balloon 26 may be formed in a substantially cylindrical shape whereby the first end region 12 and/or the second end region 14 include a rounded (e.g., curved) portion. However, this is not intended to be limiting. Rather, each of the second expandable balloon 20, the third expandable balloon 22, the fourth expandable balloon 24 and/or the fifth expandable balloon 26 may be formed in a variety of different shapes and/or configurations.

It is contemplated that in some instances the spacing between the adjacent expandable balloons may not be uniform. In some examples, the spacing between the second expandable balloon 20, the third expandable balloon 22, the fourth expandable balloon 24 and/or the fifth expandable balloon 26 may be variable (e.g., non-uniformly spaced) around the circumference of the first expandable balloon 18.

Additionally, FIG. 1 illustrates that the expandable member 16 may be coupled to a delivery catheter 25 (e.g., core wire, tubular delivery catheter, etc.). As will be discussed in greater detail below, the delivery catheter 25 may be attached to the second end region 14 of the expandable member via a variety of attachment techniques.

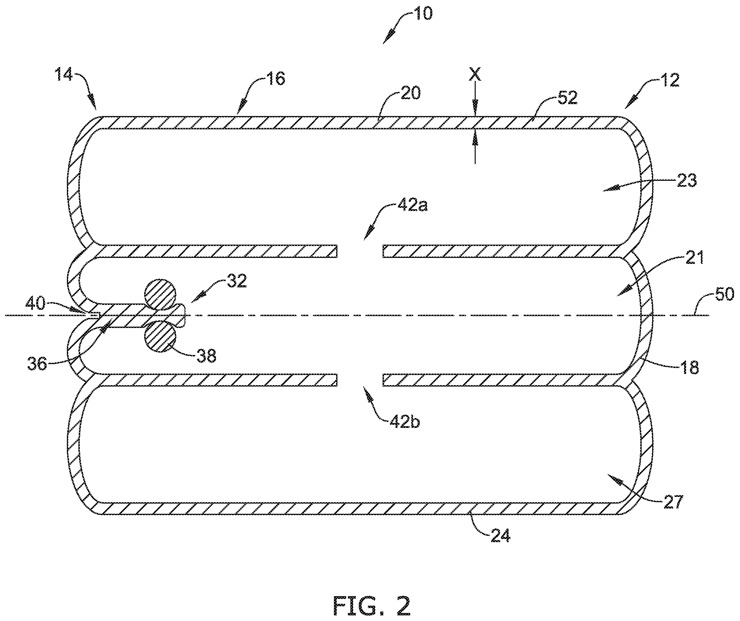

FIG. 2 illustrates a partial cross-section of the expandable member 16 illustrated in FIG. 1 taken along line 2-2. The cross-section of FIG. 2 illustrates the expandable member 16 oriented to show the second expandable balloon 20 and the fourth expandable balloon 24 positioned above and below the first expandable balloon 18. Further, FIG. 2 illustrates the first balloon 18, the second balloon 20 and the fourth balloon 24 extending longitudinally from the second end region 14 to the first end region 12 along the longitudinal axis 50.

FIG. 2 further illustrates that each of the first balloon 18, the second balloon 20 and the fourth balloon 24 include an internal inflation chamber designed to expand when filled with an inflation material. For example, the first balloon 18 includes an inflation chamber 21, the second balloon includes an inflation chamber 23 and the fourth balloon includes an inflation chamber 27.

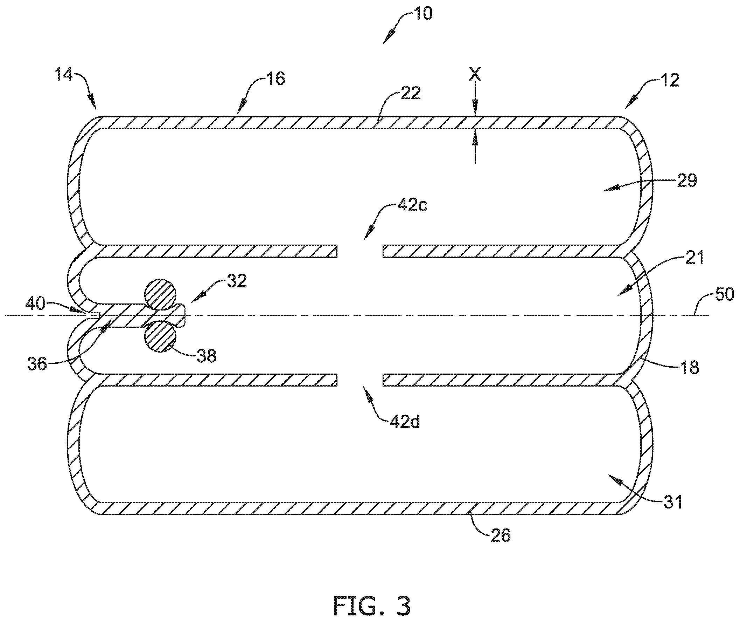

Further, FIG. 2 illustrates that expandable member 16 may include a wall 52 which forms each of the first balloon 18, the second balloon 20, the third balloon 22 (not shown in FIG. 2, but shown in FIG. 3), the fourth balloon 24 and the fifth balloon 26 (not shown in FIG. 2, but shown in FIG. 3). The wall 52 may include a uniform thickness "X." However, this is not intended to be limiting. Rather, it is contemplated that the thickness "X" of the wall 52 of the expandable member 16 may vary. For example, different portions of the first expandable balloon 18, the second expandable balloon 20, the third expandable balloon 22, the fourth expandable balloon 24 and/or the fifth expandable balloon 26 may vary in wall thickness. Varying the wall thickness in different portions of the expandable member 16 may permit the inflation characteristics of the expandable member 16 to be customized. For example, as will be discussed in greater detail below, it may be desirable to design the expandable member 16 such that some portions of the expandable member 16 expand differently (e.g., to a greater volume, length, shape, geometry, etc.) from other portions.

FIG. 2 further illustrates that, in some examples, one or more of the inflation chambers of the first balloon 18, the second expandable balloon 20, the third expandable balloon 22, the fourth expandable balloon 24 and/or the fifth expandable balloon 26 may be in fluid communication with one another. For example, FIG. 2 illustrates that the inflation chamber 21 of the first balloon 18 may be in fluid communication with the inflation chamber 23 of the second balloon and/or the inflation chamber 27 of the fourth balloon 24. For example, FIG. 2 illustrates that the expandable member 16 may include a first aperture 42a extending between the first inflation chamber 21 and the second inflation chamber 23 and a second aperture 42b extending between the first inflation chamber 21 and the third inflation chamber 27. The apertures 42a, 42b include openings in the wall 52 shared between the first balloon 18 and each of the second balloon 20 and the fourth balloon 24, respectively. As will be illustrated in greater detail below, the apertures 42a, 42b may permit inflation material to flow from the first inflation chamber 21 to the second inflation chamber 23 and the third inflation chamber 27.

As discussed above, FIG. 2 further illustrates that the expandable member 16 may include one or more inflation chambers in which in an inflation media (e.g., hydrogel beads, semi-solid materials, saline or other suitable liquids, gases, etc.) may be injected (via valve 32, for example) in order to expand the expandable member 16. As will be described in greater detail below, as an inflation media is inserted into the expandable member 16, one or more of the inflation chambers may expand, thereby permitting the expandable member 16 to seal against the tissue walls defining an opening in the left atrial appendage.

As stated above, inflation of one or more of the inflation chambers may be accomplished by inserting inflation media through the valve 32. As shown in FIG. 2, the valve 32 may be formed from the same material that forms the wall of the expandable member 16. In other words, the valve 32 may be an extension of the wall 52 of the expandable member 16. Additionally, as illustrated in FIG. 2, the valve 32 may be positioned within the first inflation chamber 21. For example, FIG. 2 illustrates that the valve 32 may extend (e.g., project) into first inflation chamber 21.

The valve 32 may include an inflation lumen 36 which may be designed to allow a secondary medical device to be inserted therethrough. As shown in FIG. 2, the inflation lumen 36 may be aligned with the longitudinal axis 50 of the expandable member 16. FIG. 2 shows the inflation lumen 36 in a closed configuration such that it would prevent inflation media (not shown in FIG. 2) from passing back through the valve 32. As shown in FIG. 2, in some examples the valve 32 may be maintained in a closed configuration via a torus-shaped mechanical gasket 38. For simplicity purposes, the gasket 38 may be referred to as an "O-ring" in the remaining discussion.

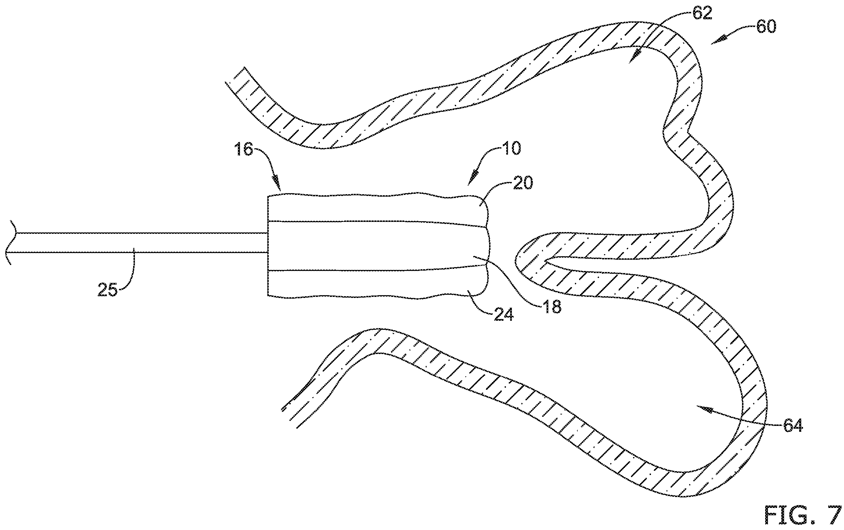

It can be appreciated that the O-ring 38 may be formed from a material (e.g., rubber, elastomer, etc.) which permits it to compress radially inwardly. As shown in FIG. 2, the O-ring 38 may be positioned around the valve 32 such that the O-ring 38 compresses the lumen 36 of valve 32 shut. However, the O-ring 38 must also permit the lumen 36 to open enough for a secondary medical device to be inserted therethrough (for inflation of the expandable member 16 as described above). Therefore, in some examples the O-ring 38 may designed to stretch and allow an inflation device access to the first inflation chamber 21 while also exerting sufficient radially inward force to maintain the lumen 36 in a closed configuration once the first inflation chamber 21 has been inflated and after the inflation device (not shown in FIG. 2) is removed from the lumen 36 (inflation of the expandable member 16 will be discussed with respect to FIGS. 7-9 below).

As will be discussed in greater detail below, the occlusive member 10 may be coupled to a delivery system in a variety of ways. Further, a component of the delivery system may also function as a secondary medical device utilized to inflate the expandable member 16. FIG. 2 illustrates an attachment region 40 which may be utilized to attach the delivery system to the occlusive member 10. Attachment region 40 may be include a variety of features which permit attachment to a delivery system. For example, attachment region 40 may include threads which mate with a threaded region on a delivery catheter (not shown in FIG. 2). In other examples, the attachment region 40 may be designed such that it forms a "press-fit" with a distal end region of a delivery catheter. Other methods of attaching the occlusive device 10 to the delivery catheter may include a ratcheting mechanism, break-away mechanisms, detent lock, spring lock, single-piece coupling, two-piece coupling, or combinations thereof.

FIG. 3 illustrates a partial cross-section of the expandable member 16 illustrated in FIG. 1 taken along line 3-3. The cross-section of FIG. 3 illustrates the expandable member 16 oriented to show the third expandable balloon 22 and the fifth expandable balloon 26 positioned above and below the first expandable balloon 18. Further, FIG. 3 illustrates the first balloon 18, the third balloon 22 and the fifth balloon 26 extending longitudinally from the second end region 14 to the first end region 12 along the longitudinal axis 50.

FIG. 3 further illustrates that each of the first balloon 18, the third balloon 22 and the fifth balloon 26 include an internal inflation chamber designed to expand when filled with an inflation material. For example, the first balloon 18 includes an inflation chamber 21, the third balloon 22 includes an inflation chamber 29 and the fifth balloon 26 includes an inflation chamber 31.

Further, FIG. 3 illustrates that expandable member 16 may include a wall 52 which defines each of the first balloon 18, the second balloon 20 (discussed with respect to FIG. 2), the third balloon 22, the fourth balloon 24 (discussed with respect to FIG. 2) and the fifth balloon 26. The wall 52 may include a uniform thickness "X." However, this is not intended to be limiting. Rather, it is contemplated that the thickness of the wall 52 of the expandable member 16 may vary in thickness.

As discussed above, in some examples, one or more of the inflation chambers of the first balloon 18, the second expandable balloon 20, the third expandable balloon 22, the fourth expandable balloon 24 and/or the fifth expandable balloon 26 may be in fluid communication with one another. For example, FIG. 3 illustrates that the inflation chamber 21 of the first balloon 18 may be in fluid communication with the fourth inflation chamber 29 of the third balloon and/or the fifth inflation chamber 31 of the fifth balloon 26. For example, FIG. 3 illustrates that the expandable member 16 may include a third aperture 42c extending between the first inflation chamber 21 and the fourth inflation chamber 29 and a fourth aperture 42d extending between the first inflation chamber 21 and the fifth inflation chamber 31. The apertures 42c, 42d include openings in the wall 52 shared between the first balloon 18 and each of the third balloon 22 and the fifth balloon 26, respectively. As will be illustrated in greater detail below, the apertures 42c, 42d may permit inflation material to flow from the first inflation chamber 21 to the fourth inflation chamber 29 and the fifth inflation chamber 31. As described above, FIG. 3 also illustrates the valve 32 including the O-ring 38 compressing the lumen 36 shut.

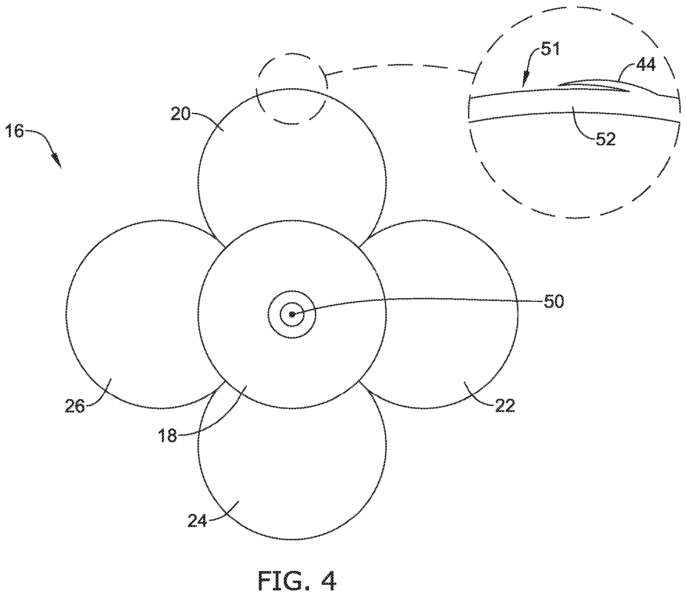

FIG. 4 illustrates an end view of the expandable member 16 described in FIGS. 1-3. FIG. 4 illustrates the second balloon 20, the third balloon 22, the fourth balloon 24 and the fifth balloon 26 circumferentially spaced around the first central balloon 18. It can be appreciated that FIG. 4 may illustrate the expandable member 16 in an unexpanded configuration.

FIG. 4 further illustrates the valve member 32 center along the longitudinal axis 50 of the expendable member 16 (e.g., the valve member 32 may be positioned along the second end region 14 as described above). The valve 32 may be utilized as an access aperture to insert a secondary medical device (not shown). The secondary medical device may be utilized to inject a fluid material into the expandable member 16.

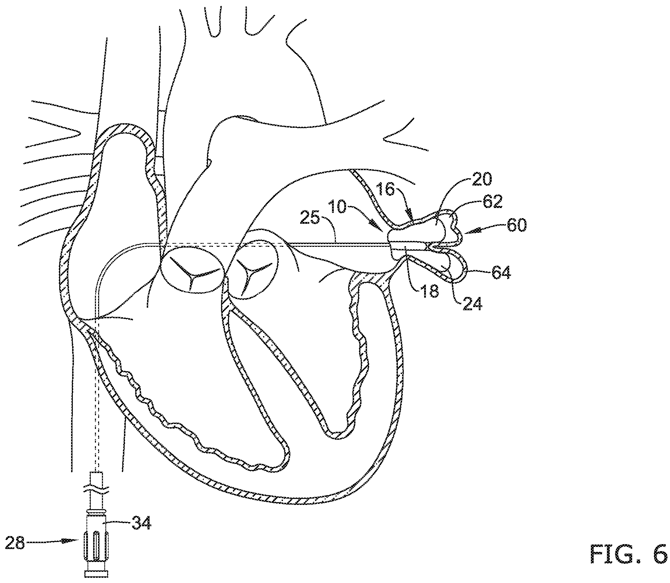

The detailed view of FIG. 4 further illustrates that any of the occlusive device examples described herein may include one or more anchor members 44 disposed along an outer surface 51 the expandable member 16. The anchor members 44 may be formed from same material as the wall 52 of the expandable member 16. It can be appreciated that in some examples the expandable member 16 may be affixed to a left atrial appendage by the one or more anchoring members 44. For example, when the expandable member 16 is positioned adjacent the inner surface of the left atrial appendage (as shown in FIG. 6), the anchor members 44 may extend radially outward from the outer surface 51 of the expandable member 16 and contact the tissue of the left atrial appendage thereby anchoring the occlusive implant in a fixed position. While FIG. 4 illustrates a single anchor member 44 positioned along expandable member 16, this is not intended to be limiting. Rather, the occlusive device may include 1, 2, 3, 4, 5, 6, 7, 8 or more anchor members 44. The anchor members 44 may improve the ability of the occlusive device to grip and maintain its position when positioned within the left atrial appendage.

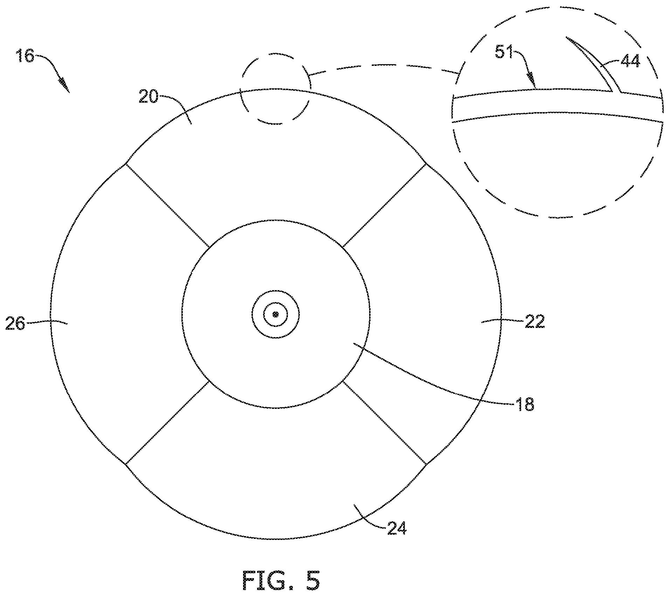

In some examples it may be desirable to design the expandable member 16 such that the one or more anchor members 44 may shift from an unextended configuration when the expandable member 16 is in an unexpanded configuration to an extended configuration when the expandable member 16 is in an expanded configuration. For example, FIG. 4 illustrates that the anchoring member 44 may lie substantially flush along the outer surface 51 of the expandable member 16 when the expandable member 16 is in an unexpanded configuration. However, FIG. 5 illustrates that the anchoring member 44 may "lift off" and project away from the outer surface 51 of the expandable member 16 when the first balloon 18, the second balloon 20, the third balloon 22, the fourth balloon 24 and/or the fifth balloon are in an expanded configuration.

FIG. 6 illustrates that the occlusive implant 10 may be inserted and advanced through a body lumen via an occlusive implant delivery system 28. FIG. 6 further illustrates the occlusive implant 10 positioned within the left atrial appendage 60. As discussed above, in some instances the occlusive implant 10 may be positioned within a bifurcated left atrial appendage. For example, FIG. 6 illustrates that the bifurcated let atrial appendage 60 may include a first lobe 62 and a second lobe 64. Additionally, FIG. 6 illustrates that the occlusive device 10 has been inflated (e.g., at least partially deployed) within the left atrial appendage 60 such that the second balloon 20 is positioned within the first lobe 62 while the fourth balloon 24 is positioned within the second lobe 64. Additionally, FIG. 6 illustrates the first balloon 18 positioned adjacent the second balloon 20 and the fourth balloon 24 as described above.

In some instances, an occlusive implant delivery system 28 may include a delivery catheter 25 which is guided toward the left atrium via various chambers and lumens of the heart (e.g., the inferior vena cava, the superior vena cava, the right atrium, etc.) to a position adjacent the left atrial appendage 60. The delivery system 28 may include a hub member 34 coupled to a proximal region of the delivery catheter 25. The hub member 34 may be manipulated by a clinician to direct the distal end region of the delivery catheter 25 to a position adjacent the left atrial appendage 60. As discussed above, a proximal end of the occlusive device 10 may be configured to releasably attach, join, couple, engage, or otherwise connect to the distal end of the delivery catheter 25. In some embodiments, a proximal end region of the occlusive device 10 may include a threaded insert coupled thereto. In some embodiments, the threaded insert may be configured to and/or adapted to couple with, join to, mate with, or otherwise engage a threaded member disposed at the distal end of the delivery catheter 25. Other means of releasably coupling and/or engaging the proximal end of the occlusive device 10 to the distal end of the delivery catheter 25 are also contemplated. Further, in some examples the delivery catheter 25 may include an inflation lumen (not show) designed to permit inflation media to pass into the occlusive device 10 (as described above). For example, in some examples, the distal end of the delivery catheter 25 may include a needle designed to be inserted through the valve 32 (discussed above).

FIGS. 7-9 illustrate the example occlusive device 10 (described above) being positioned and deployed in a bifurcated left atrial appendage 60 (e.g., a left atrial appendage including a first lobe 62 and a second lobe 64). As discussed above, in some examples, the occlusive device 10 may be configured to shift between a collapsed configuration and an expanded configuration. For example, in some instances, the occlusive implant may be in a collapsed configuration during delivery via an occlusive device delivery system, whereby the occlusive device expands to an expanded configuration once deployed from the occlusion implant delivery system.

FIG. 7 shows the occlusive device 10 including an expandable member 16 including the first balloon 18, the second balloon 20 and the fourth balloon 24. It can be appreciated that the expandable member 16 may include the third balloon 22 and the fifth balloon 26 as described above, but that the third balloon 22 and the fifth balloon 26 are hidden from view in FIGS. 7-9. Fir simplicity purposes, FIGS. 7-9 will illustrate the expansion of the first balloon 18, the second balloon 22 and the fourth balloon 24, however, the following discussion may be applied to all the balloons of the occlusive device 10 (including the third balloon 22 and the fifth balloon 26). For example, when being deployed all the balloons may be inflated and expanded to varying extents.

Further, FIG. 7 illustrates that the occlusive member 10 may be detachably coupled to a delivery catheter 25. The occlusive member 10 shown in FIG. 7 may be described as being in a deflated or delivery configuration. In other words, the expandable member 16 may not contain any inflation media within any of its inflation chambers. It can be appreciated that it may be desirable to maintain the occlusive member 10 in a collapsed configuration when delivering the occlusive member 10 to the target site (e.g., openings in the bifurcated left atrial appendage 60). A collapsed configuration may permit the occlusive member 10 to more easily track through tortuous vasculature as a clinician directs the device to the target site.

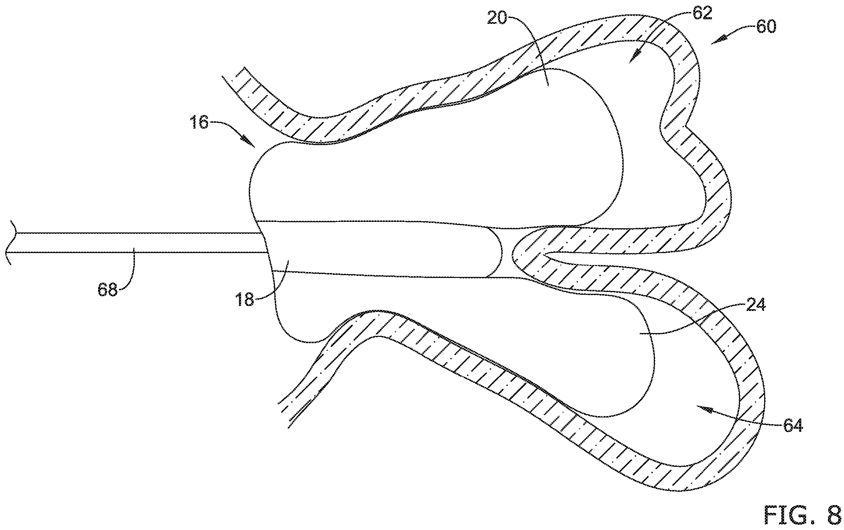

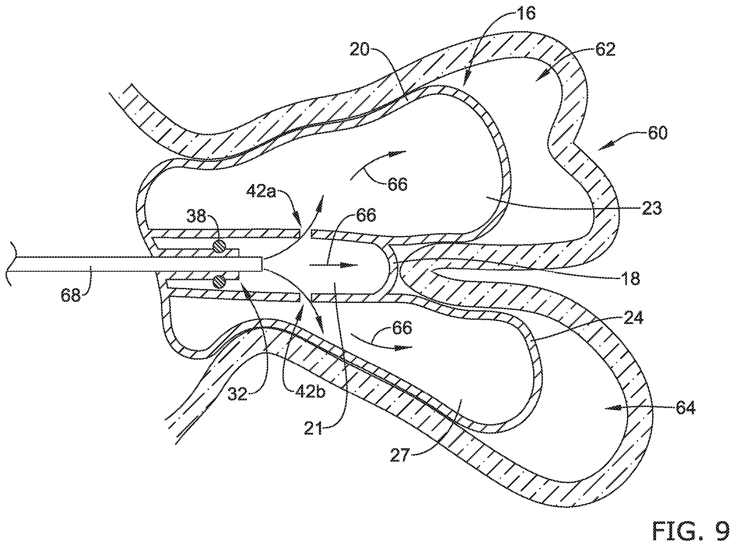

FIG. 8 illustrates an example first stage in deployment of the occlusive member 10. Initially, FIG. 8 illustrates that the delivery catheter 25 has been replaced by an example inflation catheter 68. As will be shown in FIG. 9, the distal end of the inflation catheter may extend through the valve 32 described above. Further, FIG. 8 shows the first balloon 18, the second balloon 20 and the fourth balloon 24 expanded to a larger volume (e.g., diameter) as compared with the non-expanded configuration illustrated in FIG. 7. It can be appreciated that inflation media has been injected via the inflation catheter 68 into the inner chambers of the first balloon 18, the second balloon 20 and the fourth balloon 24, whereby the inflation media shifts the expandable member 16 from the deflated configuration (shown in FIG. 7) to the partially-inflated configuration shown in FIG. 8.

Additionally, as described above, FIG. 8 illustrates that the second balloon 22 and the fourth balloon 24 have been elongated to a greater extent than the first balloon 18. In other words, the second balloon 22 and the fourth balloon 24 may be designed such that they expand to fill (e.g., extend into) the first lobe 62 and the second lobe 64 of the bifurcated atrial appendage 60, while the first balloon 18 is prevented from expanding to the extent of the second balloon 22 and the fourth balloon 24. Additionally, it can be appreciated that that as the expandable member 16 inflates radially outward, the anchor members 44 (not shown, but discussed above) may approach and may contact the inner surface (e.g., the tissue wall) of the left atrial appendage 60.

FIG. 9 is a cross-sectional view of the occlusive member 10 described in FIG. 8. FIG. 9 illustrates that the O-ring 38 has expanded to permit the distal end region of the inflation catheter 68 to extend through the valve 32. Additionally, FIG. 9 shows an inflation material 66 being injected with the inflation catheter into the first balloon inflation chamber 21. Further, FIG. 9 illustrates the inflation material 66 passing through the first aperture 42a and the second aperture 42b such that the inflation material 66 passes into the second inflation chamber 23 (of the second balloon 20) and the third inflation chamber 27 (of the fourth balloon 24). It can further be appreciated that a portion of the inflation material 66 may remain in the first inflation chamber 21 of the first balloon 18.

FIG. 9 illustrates that the expandable member 16 may be compliant and, therefore, substantially conform to and/or be in sealing engagement with the shape and/or geometry of the first lobe 62 and/or the second lobe 64 of the bifurcated left atrial appendage 60 while in the inflated (e.g., expanded) configuration. In some embodiments, the occlusive device 10 may expand to a size, extent, or shape different from a maximum unconstrained extent, as determined by the surrounding tissue and/or lateral wall of the bifurcated left atrial appendage 60.

As can be appreciated from FIGS. 8-9, continued inflation of the expandable member 16 beyond the partially inflated state shown in FIG. 7 may permit the expandable member 16 to expand and conform to the specific geometry of the inner surface of the first lobe 62 and/or the second lobe 64 of the bifurcated left atrial appendage 60. In other words, as inflation media is added to the expandable member 16, the expandable member 16 may fill and/or seal gaps in the opening of first lobe 62 and/or the second lobe 64 of the bifurcated left atrial appendage 60 which may not have been sealed while the occlusive device 10 was partially inflated (as shown in FIG. 7). It can be appreciated that the flexible material used to construct the expandable member 16 may stretch, conform and directly oppose the folded curvature of the inner surface of first lobe 62 and/or the second lobe 64 of the bifurcated left atrial appendage 60. For example, FIG. 9 shows the expandable member 16 expanded such that the expandable member 16 is contacting the curved inner surface of the left atrial appendage 60, thereby sealing the opening of the left atrial appendage 60.

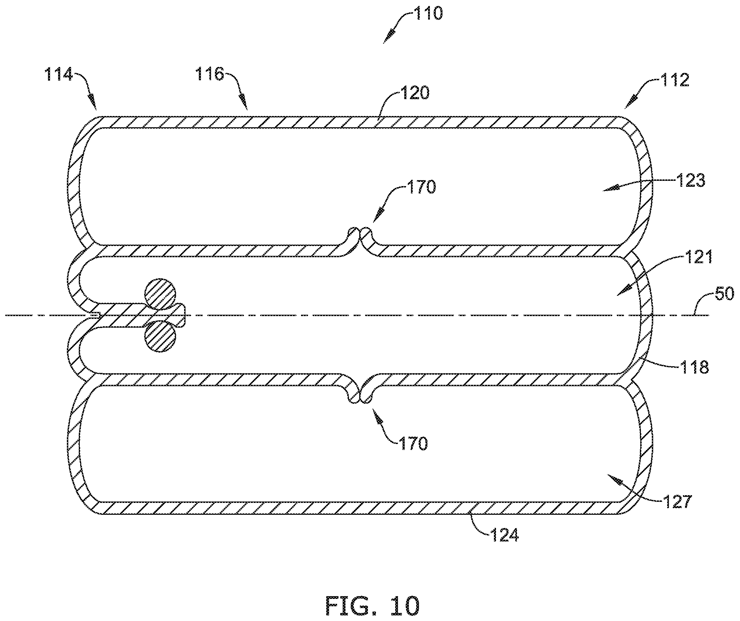

FIG. 10 illustrates another example occlusive device 110. The occlusive device 110 may be similar in form and function to other occlusive devices described herein. For example, the occlusive device 10 may include an expandable member 116 having a first balloon 118, a second balloon 120 and a fourth balloon 124, whereby the first, second and fourth balloons 118, 120, 124 extend along the longitudinal axis 50 from a first end 112 to a second end 114 of the expandable member 116. In addition, the expandable member 116 may include a third balloon and fifth balloon as described above.

Additionally, FIG. 10 illustrates that the expandable member 116 may include one or more valves 170 positioned between a first inflation chamber 121 of the first balloon 118 and a second inflation chamber 123 of the second balloon and/or a fourth inflation chamber 127 of the fourth balloon 124. In some examples, the valves 170 may include one-way valves which permit fluid to flow from the first inflation chamber 121 to the second and fourth inflation chambers 123,127, respectively, while preventing fluid to flow from the second and fourth inflation chambers 123,127, respectively, back into the first inflation chamber 121.

Further, in some examples the valves 170 may be designed to open after a threshold inflation pressure has been attained in the first inflation chamber. For example, in some instances, when the first inflation chamber 121 is filled with inflation material to a threshold inflation pressure, one or more of the valves 170 may open, thereby permitting the inflation material to pass through the valves 170 into the second inflation chamber 123 and/or the fourth inflation chamber 127.

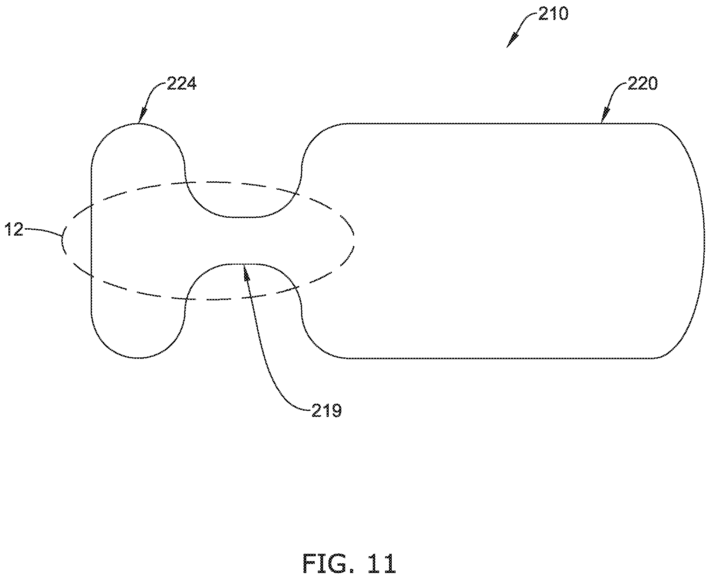

FIG. 11 illustrates another example occlusive device 210. The occlusive device 210 may include a first inflation balloon 224 positioned proximal to a second inflation balloon 220. The second inflation balloon 220 may be different in shape (e.g., length and/or width) than the first inflation balloon 224. Additionally, a transition region 219 may extend between the first inflation balloon 224 and the second inflation balloon 220. In some examples, the first inflation balloon 224 may be positioned adjacent an opening to the left atrial appendage (e.g., the transition region 219 maybe designed to nest with the ostium of the left atrial appendage) while the second inflation balloon 220 may be positioned within one or more cavities defining a bifurcated left atrial appendage.

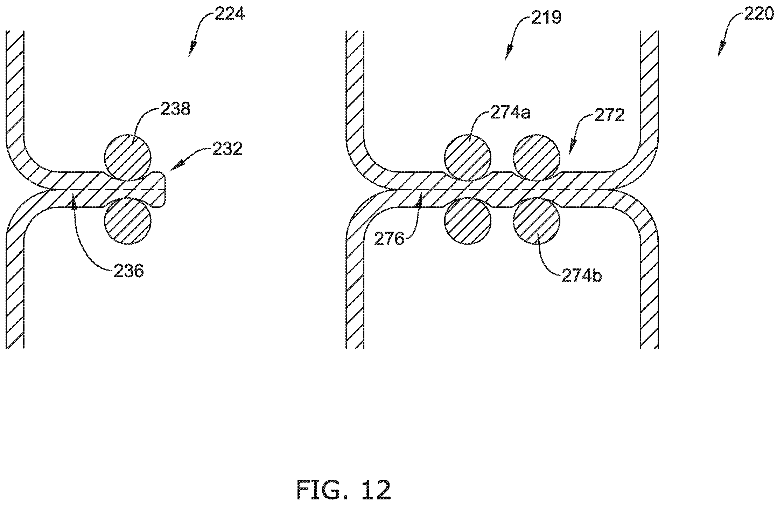

FIG. 12 illustrates a cross section of taken along the dashed portion shown in FIG. 11. FIG. 12 illustrates that the first inflation balloon 224 may include a valve member 232 extending into the inflation chamber of the first inflation balloon 224. The valve member 232 may be similar in form and function to the valve 32 described above. For example, the valve 232 may include an inflation lumen 236 which may allow a secondary medical device to be inserted therethrough. FIG. 12 shows the inflation lumen 36 in a closed configuration such that it would prevent inflation media (not shown in FIG. 12) from passing back through the valve 232.

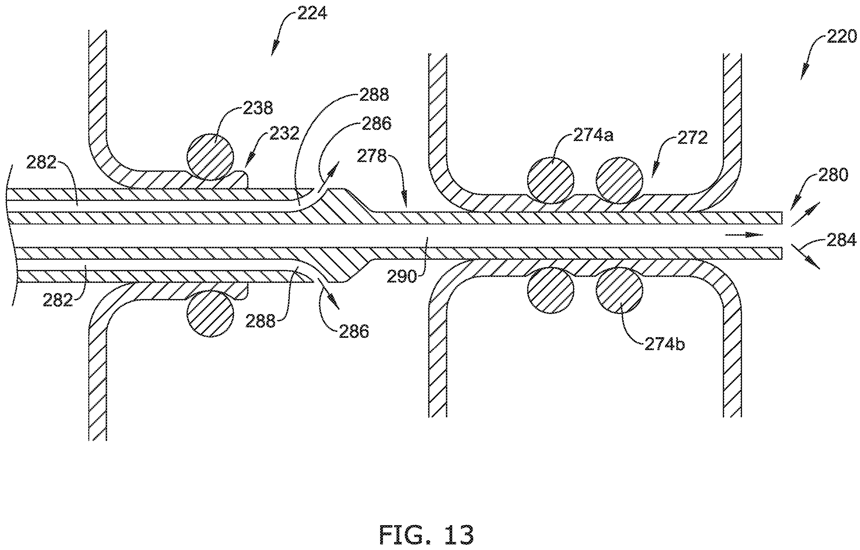

As shown in FIG. 12, in some examples the valve 232 may be maintained in a closed configuration via a torus-shaped O-ring 238. As described above, it can be appreciated that the O-ring 238 may be formed from a material (e.g., rubber, elastomer, etc.) which permits it to compress radially inwardly. As shown in FIG. 12, the O-ring 238 may be positioned around the valve 232 such that the O-ring 238 compresses the lumen 236 of valve 232 shut. However, the O-ring 238 must also permit the lumen 236 to open enough for a secondary medical device to be inserted therethrough (for inflation of the first inflation balloon 224 described above). Therefore, in some examples the O-ring 238 may designed to stretch and allow an inflation device access to the first inflation chamber of the first inflation balloon 224 while also exerting sufficient radially inward force to maintain the lumen 236 in a closed configuration once the first balloon inflation chamber has been inflated and after the inflation device (not shown in FIG. 2) is removed from the lumen 236 (inflation of the first inflation balloon 224 and the second inflation balloon 220 will be discussed with respect to FIG. 13 below).

FIG. 12 further illustrates that the occlusive device 210 may include a second valve member 272 positioned within the transition section 219. The second valve member 272 may include an inflation lumen 276 which may be designed to allow a secondary medical device to be inserted therethrough. FIG. 12 shows the inflation lumen 276 in a closed configuration such that it would prevent inflation media (not shown in FIG. 12) from passing back through the valve 276 from the second inflation balloon 220 to the first inflation balloon 224.

As shown in FIG. 12, in some examples the valve 272 may be maintained in a closed configuration via one or more torus-shaped O-rings 274a, 274b. As described above, it can be appreciated that the O-rings 274a, 274b may be formed from a material (e.g., rubber, elastomer, etc.) which permit them to compress radially inwardly. As shown in FIG. 12, the O-rings 274a, 274b may be positioned around the valve 272 such that the O-rings 274a, 274b compresses the lumen 276 of valve 272 shut. However, the O-rings 274a, 274b must also permit the lumen 276 to open enough for a secondary medical device to be inserted therethrough (for inflation of the second inflation balloon 220 described above). Therefore, in some examples the O-rings 274a, 274b may designed to stretch and allow an inflation device access to the inflation chamber of the second inflation balloon 220 while also exerting sufficient radially inward force to maintain the lumen 276 in a closed configuration once the second balloon inflation chamber has been inflated and after the inflation device (not shown in FIG. 12) is removed from the lumen 276 (inflation of the first inflation balloon 224 and the second inflation balloon 220 will be discussed with respect to FIG. 13 below).

FIG. 13 illustrates an example inflation catheter 278 positioned within the occlusive device 210 such that it may inflate the first inflation balloon 224 and/or the second inflation balloon 220. As illustrated in FIG. 13, the inflation device 278 may be inserted through both the first valve 232 (via expansion of the O-ring 238) and the second valve 276 (via expansion of the O-rings 274a, 274b) such that a port 280 located along the distal end region 280 of the inflation catheter 278 may extend into the inflation chamber of the second balloon 220. As illustrated in FIG. 12, the port 280 may correspond to a distal opening of a centrally-located lumen 290 passing through the inflation catheter 278.

While the above example illustrates that O-rings 238 may be utilized to seal the first valve 232 and/or the second valve 276, this is not intended to me limiting. Rather, it is contemplated that, in some examples, the O-rings 238, 274a, 274b may not be necessary to seal the first valve 232 and/or the second valve 276. Rather, in some examples, the first valve 232 and/or the second valve 276 may include sufficient radially inward compressive strength to seal around an inflation catheter 278 inserted therethrough.

Additionally, FIG. 13 illustrates that the inflation catheter 278 may include one or more additional ports 288 positioned proximal to the port 280. In particular, the ports 288 may be positioned such that when the port 280 is positioned within the inflation chamber of the second balloon 220, the ports 288 may be positioned within the inflation chamber of the first balloon 224. As illustrated in FIG. 13, the ports 288 may correspond to openings of one or more lumens 282 passing through the inflation catheter 278. The lumens 282 may be radially outward of the centrally positioned lumen 290.

FIG. 13 further illustrates that, in some examples, inflation material 284 may pass through the first lumen 290 and exit port 280 to inflate the inner balloon chamber of the second balloon 220. In some examples, inflation material 286 may simultaneously pass through the lumens 282 and out ports 288 to inflate the inner balloon chamber of the first balloon 224.

However, in other examples, the inflation chamber of the first balloon 224 may be inflated in sequence with the inflation chamber of the second balloon 220. For example, the inflation chamber 220 of the second balloon may be inflated via the lumen 290 followed by inflation of the inflation chamber of the first balloon 224. Further yet, it can be appreciated that the inflation chamber 220 of the second balloon may be inflated via lumen 290 and port 280, whereby a clinician may then withdraw the inflation device 278 proximally through valve 272 such that the O-rings 274a, 274b collapse and seal the valve 272. After the valve 272 is seal (and the inflation chamber of the second balloon is pressurized) the clinician may continue to inject inflation material through the lumen 290 and port 280 to inflate the inner chamber of the first balloon 224. After the first balloon 224 is inflated, the clinician may remove the inflation catheter 278 from the first valve 232, thereby sealing the inner chamber of the first balloon 224.

It can be appreciated that this inflation technique may permit the first balloon 224 and the second balloon 220 may be inflated to different pressures. It can be further appreciated that the first balloon 224 and the second balloon 220 may be inflated with different materials. For example, in some examples the first balloon 224 may be inflated with saline while the second balloon 220 may be inflated with hydrogel. In other examples, the first balloon 224 may be inflated with hydrogel while the second balloon 220 may be inflated with saline. Additionally, other inflation materials are contemplated, as described below.

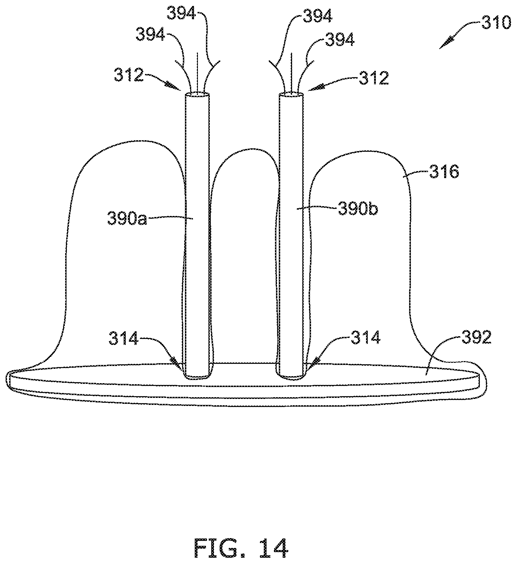

FIG. 14 illustrates another occlusive device 310. The occlusive device 310 may include a base 392 coupled (e.g., attached, secured, etc.) to a first end 314 of a first attachment arm 390a and a first end 314 of a second attachment arm 390b. The occlusive device 310 may also include an expandable member 316 disposed adjacent to and/or engaged with the base 392, the first attachment arm 390a and/or the second attachment arm 390b. In some examples, the expandable member 316 may fully or partially surround the base 392, the first attachment arm 390a and/or the second attachment arm 390b.

While FIG. 14 illustrates a two attachment arms 390a, 390b, this is not intended to be limiting. Rather, it is contemplated that the occlusive device 310 may include more or less than two attachment arms. For example, the occlusive device 310 may include 1, 2, 3, 4, 5, 6, 7, 8, 9, 10 or more attachment arms.

As shown in FIG. 14, each of the first attachment arm 390a and the second attachment arm 390b may extend away from the base 392. For example, each of the first attachment arm 390a and the second attachment arm 390b may extend substantially straight away from the base 392. Additionally, each of the first attachment arm 390a and the second attachment arm 390b may include a second end 312 positioned opposite to the first end 314 of each of the first attachment arm 390a and the second attachment arm 390b, respectively.

Additionally, FIG. 14 illustrates that each of the first attachment arm 390a and the second attachment arm 390b may include or more fixation members 394 coupled to the second end 312 of each of the first attachment arm 390a and/or the second attachment arm 390b. The fixation members 394 may include a barb, hook, projection, prong, spur, etc. While FIG. 14 illustrates three fixation members 394 positioned along each of the first attachment arm 390a and the second attachment arm 390b, this is not intended to be limiting. Rather, the occlusive device 310 may include 1, 2, 3, 4, 5, 6, 7, 8 or more fixation members 394 disposed along each of the first attachment arm 390a and/or the second attachment arm 390b.

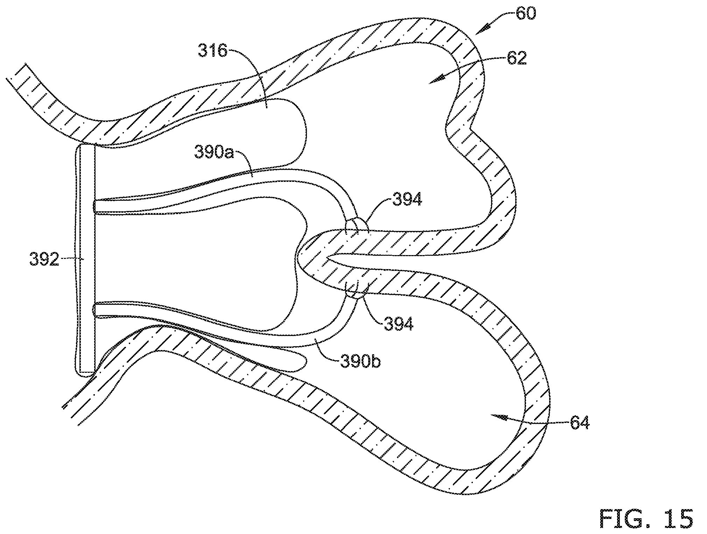

FIG. 15 illustrates that the fixation members 394 may be designed to extend radially outward from each of the first attachment arm 390a and/or the second attachment arm 390b and contact the tissue of a bifurcated left atrial appendage 60, thereby anchoring the occlusive implant 310 in a fixed position within the left atrial appendage 60. For example, FIG. 15 illustrates that the first attachment arm 390a may extend within and anchor along an inner surface of the first lobe 62 of the bifurcated atrial appendage 60 and that the second attachment arm 390b may extend within and anchor along an inner surface of the second lobe 64 of the bifurcated atrial appendage 60. It can be appreciated that the anchor members 394 may improve the ability of the occlusive device 310 to grip and maintain its position when positioned within the left atrial appendage 60.

Additionally, FIG. 15 illustrates that each of the first attachment arm 390a and/or the second attachment arm 390b may secure the occlusive device 310 within the left atrial appendage 60 such that the base 392 seals the opening to the left atrial appendage 60. In some examples, the attachment arms 390a, 390b may include a spring or similar structure to "pull" the base 392 into a secure fit within the opening of the left atrial appendage 60.

FIG. 15 further illustrates the expandable member 316 after having been inflated (e.g., at least partially deployed) within the left atrial appendage 60 such that the expandable member 316 is positioned within the first lobe 62 and/or the second lobe 64 of the bifurcated atrial appendage. The expansion of the expandable member 316 may be similar to the expansion of other example expandable members described herein.

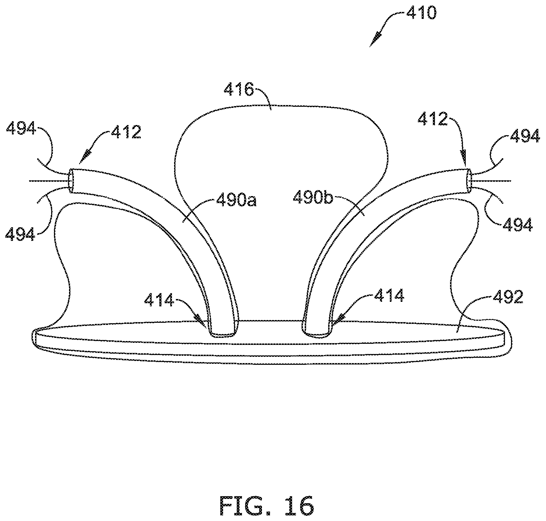

FIG. 16 illustrates another occlusive device 410. The occlusive device 410 may include a base 492 coupled (e.g., attached, secured, etc.) to a first end 414 of a first attachment arm 490a and a first end 414 of a second attachment arm 490b. The occlusive device 410 may also include an expandable member 416 disposed adjacent to and/or engaged with the base 492, the first attachment arm 490a and/or the second attachment arm 490b. In some examples, the expandable member 416 may fully or partially surround the base 492, the first attachment arm 490a and/or the second attachment arm 490b.

As shown in FIG. 16, each of the first attachment arm 490a and the second attachment arm 490b may extend away from the base 492. For example, each of the first attachment arm 490a and the second attachment arm 490b may extend in a curved path away from the base 492 (e.g., each of the first attachment arm 490a and/or the second attachment arm 490b may flare outward, away from another one). Additionally, each of the first attachment arm 490a and the second attachment arm 490b may include a second end 412 positioned opposite to the first end 414 of each of the first attachment arm 490a and the second attachment arm 490b, respectively.

Additionally, FIG. 16 illustrates that each of the first attachment arm 490a and the second attachment arm 490b may include or more fixation members 494 coupled to the second end 412 of each of the first attachment arm 490a and/or the second attachment arm 490b. The fixation members 494 may include a barb, hook, projection, prong, spur, etc. While FIG. 16 illustrates three fixation members 494 positioned along each of the first attachment arm 490a and the second attachment arm 490b, this is not intended to be limiting. Rather, the occlusive device 410 may include 1, 2, 3, 4, 5, 6, 7, 8 or more fixation members 494 disposed along each of the first attachment arm 490a and/or the second attachment arm 490b.

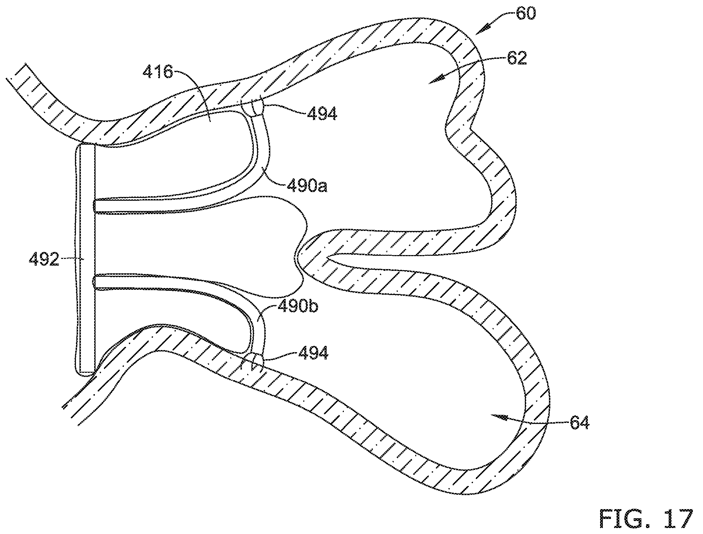

FIG. 17 illustrates that the fixation members 494 may be designed to extend radially outward from each of the first attachment arm 490a and/or the second attachment arm 490b and contact the tissue of a bifurcated left atrial appendage 60, thereby anchoring the occlusive implant 410 in a fixed position within the left atrial appendage 60. For example, FIG. 17 illustrates that the first attachment arm 490a may curve outward and attach along an inner surface of the first lobe 62 of the bifurcated atrial appendage 60 while the second attachment arm 490b may curve in an opposite direction and attach along an inner surface of the second lobe 64 of the bifurcated atrial appendage 60. It can be appreciated that the fixation members 494 may improve the ability of the occlusive device 410 to grip and maintain its position when positioned within the left atrial appendage 60.

Additionally, FIG. 17 illustrates that each of the first attachment arm 490a and/or the second attachment arm 490b may secure the occlusive device 410 within the left atrial appendage 60 such that the base 492 seals the opening to the left atrial appendage 60. In some examples, the attachment arms 490a, 490b may include a spring or similar structure to "pull" the base 492 into a secure fit within the opening of the left atrial appendage 60.

FIG. 17 further illustrates the expandable member 416 after having been inflated (e.g., at least partially deployed) within the left atrial appendage 60 such that the expandable member 416 is positioned within the first lobe 62 and/or the second lobe 64 of the bifurcated atrial appendage. The expansion of the expandable member 416 may be similar to the expansion of other example expandable members described herein.

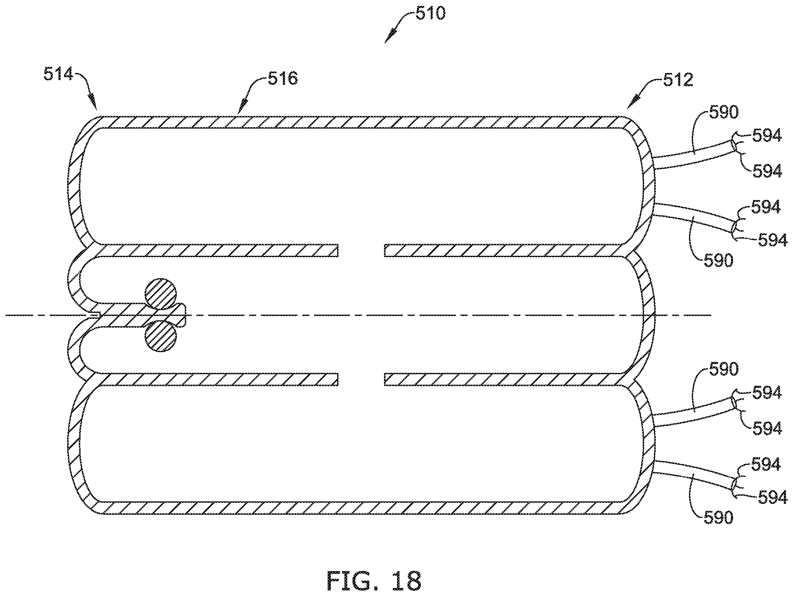

FIG. 18 illustrates another example occlusive implant 510. The occlusive implant 510 may be similar in form and function to other example occlusive implants described herein. For example, the occlusive implant 510 may be similar in form and function to the occlusive implant 10 described above with respect to FIGS. 1-6. For example, the occlusive implant 10 may include a first end region 512 and a second end region 514. As will be discussed in greater detail below, the first end region 512 may include the portion of the occlusive implant 510 which extends farthest into a left atrial appendage, while the second end region 514 may include the portion of the occlusive implant 510 which is positioned closer to an opening of the left atrial appendage.

The occlusive implant 510 may include an expandable member 516. The expandable member 516 may also be referred to as an expandable balloon 516. The expandable member 516 may be formed from a highly compliant material (e.g., "inflation material") which permits the expandable member 516 to expand from a first unexpanded (e.g., deflated, collapsed, delivery) configuration to a second expanded (e.g., inflated, delivered) configuration. In some examples, one or more portions of the expandable balloon 516 may be inflated to pressures from about 6895 Pa to 1379000 Pa (1 psi to about 200 psi). It can be appreciated that the outer diameter of the occlusive implant 510 may be larger in the expanded configuration versus the unexpanded configuration. Example materials used for the inflation material may be hydrogel beads (or other semi-solid materials), saline, etc.

In some examples, the expandable member 516 may be constructed from silicone or a low-durometer polymer, however, other materials are contemplated. Additionally, the expandable member 516 may be impermeable to blood and/or other fluids, such as water. In some embodiments, the expandable member 516 may include a woven, braided and/or knitted material, a fiber, a sheet-like material, a metallic or polymeric mesh, or other suitable construction. Further, in some embodiments, the expandable member 516 may prevent thrombi (e.g., blood clots, etc.) originating in the left atrial appendage from passing through the occlusive device 510 and into the blood stream. In some embodiments, the occlusive device 510 may promote endothelial growth after implantation, thereby effectively removing the left atrial appendage from the patient's circulatory system.

FIG. 18 further illustrates that, in some examples, the occlusive implant 510 may include one or more attachment arms 590 extending away from the first end region 512. Similar to that described above with respect to FIG. 14 and FIG. 16, each of the attachment arms 590 may include or more fixation members 594 coupled to the end of each of the attachment arms 590. The fixation members 594 may include a barb, hook, projection, prong, spur, etc. While FIG. 18 illustrates three fixation members 594 positioned along each of the attachment arms, this is not intended to be limiting. Rather, the occlusive device 510 may include 1, 2, 3, 4, 5, 6, 7, 8 or more fixation members 594 disposed along each of the attachment arms 590.

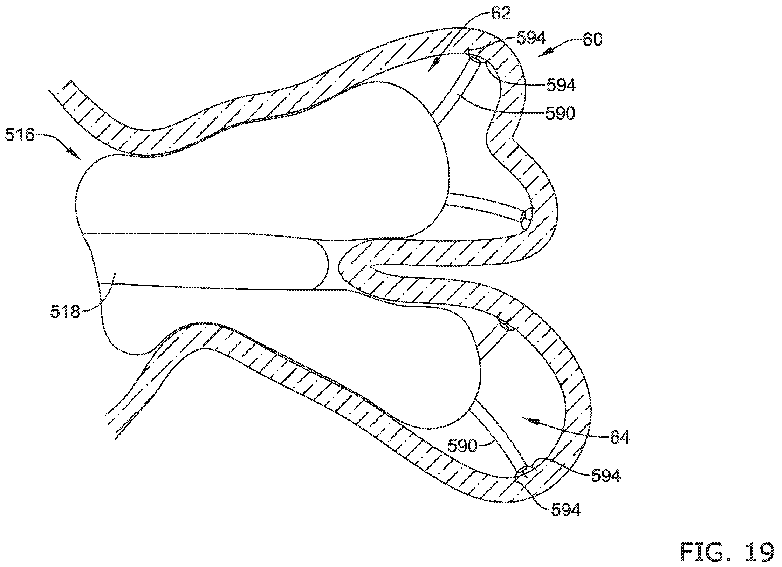

FIG. 19 illustrates that the fixation members 594 may be designed to extend radially outward from each of the attachment arms 590 and contact the tissue of a bifurcated left atrial appendage 60, thereby anchoring the occlusive implant 510 in a fixed position within the left atrial appendage 60. For example, FIG. 19 illustrates that the attachment arms 590 may extend within and anchor along an inner surface of the first lobe 62 and the second lobe 64 of the bifurcated atrial appendage 60. It can be appreciated that the anchor members 594 may improve the ability of the occlusive device 510 to grip and maintain its position when positioned within the left atrial appendage 60.

Additionally, FIG. 19 illustrates that each of the attachment arms may secure the occlusive device 510 within the left atrial appendage 60 such that the expandable member 516 seals the opening to the left atrial appendage 60. For example, FIG. 19 further illustrates the expandable member 516 after having been inflated (e.g., at least partially deployed) within the left atrial appendage 60 such that the expandable member 316 is positioned within the first lobe 62 and/or the second lobe 64 of the bifurcated atrial appendage. The expansion of the expandable member 516 may be similar to the expansion of other example expandable members described above.

The materials that can be used for the various components of the occlusive implant 10 (and variations, systems or components thereof disclosed herein) and the various elements thereof disclosed herein may include those commonly associated with medical devices. For simplicity purposes, the following discussion makes reference to the occlusive implant 10 (and variations, systems or components disclosed herein). However, this is not intended to limit the devices and methods described herein, as the discussion may be applied to other elements, members, components, or devices disclosed herein.

In some embodiments, the occlusive implant 10 (and variations, systems or components thereof disclosed herein) may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; platinum; palladium; gold; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the occlusive implant 10 (and variations, systems or components thereof disclosed herein) may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the occlusive implant 10 (and variations, systems or components thereof disclosed herein). Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the occlusive implant 10 (and variations, systems or components thereof disclosed herein). to achieve the same result.

In some embodiments, a degree of Magnetic Resonance Imaging (MRI) compatibility is imparted into the occlusive implant 10 (and variations, systems or components thereof disclosed herein). For example, the occlusive implant 10 (and variations, systems or components thereof disclosed herein) and/or components or portions thereof, may be made of a material that does not substantially distort the image and create substantial artifacts (e.g., gaps in the image). Certain ferromagnetic materials, for example, may not be suitable because they may create artifacts in an MRI image. The occlusive implant 10 (and variations, systems or components disclosed herein) or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the occlusive implant 10 (and variations, systems or components thereof disclosed herein) and/or portions thereof, may be made from or include a polymer or other suitable material. Some examples of suitable polymers may include copolymers, polyisobutylene-polyurethane, polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, polyurethane silicone copolymers (for example, ElastEon^{®} from Aortech Biomaterials or ChronoSil^{®} from AdvanSource Biomaterials), biocompatible polymers, other suitable materials, or mixtures, combinations, copolymers thereof, polymer/metal composites, and the like. In some embodiments, the sheath can be blended with a liquid crystal polymer (LCP). For example, the mixture can contain up to about 6 percent LCP.

In some embodiments, the occlusive implant 10 (and variations, systems or components thereof disclosed herein) may include a textile material. Some examples of suitable textile materials may include synthetic yarns that may be flat, shaped, twisted, textured, pre-shrunk or un-shrunk. Synthetic biocompatible yarns suitable for use in the present disclosure include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun-types. The type and denier of the yarn chosen may be selected in a manner which forms a biocompatible and implantable prosthesis and, more particularly, a vascular structure having desirable properties.

In some embodiments, the occlusive implant 10 (and variations, systems or components thereof disclosed herein) may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

While the discussion above is generally directed toward an occlusive implant for use in the left atrial appendage of the heart, the aforementioned features may also be useful in other types of medical implants where a fabric or membrane is attached to a frame or support structure including, but not limited to, implants for the treatment of aneurysms (e.g., abdominal aortic aneurysms, thoracic aortic aneurysms, etc.), replacement valve implants (e.g., replacement heart valve implants, replacement aortic valve implants, replacement mitral valve implants, replacement vascular valve implants, etc.), and/or other types of occlusive devices (e.g., atrial septal occluders, cerebral aneurysm occluders, peripheral artery occluders, etc.). Other useful applications of the disclosed features are also contemplated.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A medical device (10) for occluding a bifurcated left atrial appendage (60), comprising:
an expandable member (16) including a first central balloon (18) defining a first inflation chamber (21), a second balloon (20) defining a second inflation chamber (23) and a third balloon (24) defining a third inflation chamber (27), the second inflation chamber (23) positioned adjacent to the first inflation chamber (21), wherein the first inflation chamber (21) is in fluid communication with the second inflation chamber (23) and the third inflation chamber (27), wherein the second and third balloons (20, 24) are spaced circumferentially around the first balloon (18), and wherein the expandable member (16) is designed to shift between a first unexpanded configuration and a second expanded configuration; and
a first inflation valve member (32) extending at least partially into the first inflation chamber (21),
wherein the expandable member (16) is configured to expand and seal the opening of the left atrial appendage (60),
wherein the second balloon (20) and the third balloon (24) of the expandable member (16) are elongated to a greater extent than the first balloon (18), when the expandable member (16) is in the second expanded configuration, and
wherein the second balloon (20) is configured to expand to extend into a first lobe (62) of the left atrial appendage (60) and the third balloon (24) is configured to expand to extend into a second lobe (64) of the left atrial appendage (60).

2. The medical device (10) of claim 1, wherein the expandable member (16) is configured to inflate the first chamber (21) to a first inflation pressure, and wherein the expandable member (16) is configured to inflate the second chamber (23) to a second inflation pressure after the first chamber (21) is inflated to the first inflation pressure.

3. The medical device (10; 310) of any one of claims 1-2, further comprising one or more attachment arms (590) extending from a distal region of the second balloon and a distal region of the third balloon of the expandable member (516), the one or more attachment arms (590) including one or more projections (594) disposed thereon, wherein the one or more projections (594) are configured to engage a portion of the left atrial appendage wall.

4. The medical device (110) of any one of claims 1-3, wherein the expandable member (116) includes a first one-way valve configured to permit an inflation material to flow between the first chamber (121) and the second chamber (123), wherein the first one-way valve is configured to open when the first chamber (121) is inflated to a first threshold inflation pressure.

5. The medical device (10) of any one of claims 1-4, a fourth balloon (22) defining a fourth inflation chamber (29) in fluid communication with the first inflation chamber (21), and a fifth balloon (26) defining a fifth inflation chamber (31) in fluid communication with the first inflation chamber (21), wherein the fourth and fifth balloons (22, 26) are spaced circumferentially around the first balloon (18).

6. The medical device (310; 410; 510) of any one of claims 1-5, further comprising a projection (394; 494; 594) configured to anchor the medical device (310; 410; 510) to a target tissue site of the left atrial appendage (60).

7. The medical device (310; 410; 510) of claim 6, wherein the projection (394; 494; 594) is configured to shift between a first position and a second extended position, wherein the projection (394; 494; 594) extends radially away from an outer surface of the expandable member (316; 416; 516) in the second extended position.

8. The medical device (210) of any one of claims 1-7, further comprising a second inflation valve member (272) positioned between the first inflation chamber and the second inflation chamber.

9. The medical device (210) of claim 8, wherein the expandable member (16) is designed to engage an inflation catheter (278) having a first inflation port (280) and a second inflation port (288), and wherein the first inflation port (280) extends into the second inflation chamber through the second inflation valve member (272), and wherein the second inflation port (288) is positioned within the first inflation chamber when the first inflation port (280) is positioned within the second inflation chamber.

10. The medical device (210) of any one of claims 1-9, wherein the expandable member (16) is configured to inflate the second chamber to a first inflation pressure, and wherein the expandable member (16) is configured to inflate the first chamber to a second inflation pressure after the second chamber is inflated to the first inflation pressure, and wherein the second inflation valve member (272) is designed to maintain the first inflation pressure in the second chamber while the first inflation chamber is inflated to the second inflation pressure.

## Patentansprüche

1. Medizinische Vorrichtung (10) zum Verschließen eines zweigeteilten linken Vorhofrohrs (60), umfassend:
ein expandierbares Element (16), das einen ersten zentralen Ballon (18), der eine erste Aufblaskammer (21) definiert, einen zweiten Ballon (20), der eine zweite Aufblaskammer (23) definiert, und einen dritten Ballon (24), der eine dritte Aufblaskammer (27) definiert, aufweist, wobei die zweite Aufblaskammer (23) angrenzend an die erste Aufblaskammer (21) positioniert ist, wobei die erste Aufblaskammer (21) mit der zweiten Aufblaskammer (23) und der dritten Aufblaskammer (27) in Fluidverbindung steht, wobei der zweite und der dritte Ballon (20, 24) in Umfangsrichtung um den ersten Ballon (18) beabstandet sind, und wobei das expandierbare Element (16) dazu gestaltet ist, zwischen einer ersten nicht expandierten Auslegung und einer zweiten expandierten Auslegung zu wechseln; und
ein erstes Aufblasventilelement (32), das sich zumindest teilweise in die erste Aufblaskammer (21) erstreckt,
wobei das expandierbare Element (16) dazu ausgelegt ist, die Öffnung des linken Vorhofrohrs (60) zu expandieren und abzudichten,
wobei der zweite Ballon (20) und der dritte Ballon (24) des expandierbaren Elements (16) zu einem größeren Ausmaß als der erste Ballon (18) verlängert sind, wenn sich das expandierbare Element (16) in der zweiten expandierten Auslegung befindet, und
wobei der zweite Ballon (20) dazu ausgelegt ist, zu expandieren, um sich in einen ersten Lappen (62) des linken Vorhofrohrs (60) zu erstrecken, und der dritte Ballon (24) dazu ausgelegt ist, zu expandieren, um sich in einen zweiten Lappen (64) des linken Vorhofrohrs (60) zu erstrecken.

2. Medizinische Vorrichtung (10) nach Anspruch 1, wobei das expandierbare Element (16) dazu ausgelegt ist, die erste Kammer (21) auf einen ersten Aufblasdruck aufzublasen, und wobei das expandierbare Element (16) dazu ausgelegt ist, die zweite Kammer (23) auf einen zweiten Aufblasdruck aufzublasen, nachdem die erste Kammer (21) auf den ersten Aufblasdruck aufgeblasen wurde.

3. Medizinische Vorrichtung (10; 310) nach einem der Ansprüche 1-2, ferner umfassend einen oder mehrere Befestigungsarme (590), die sich von einem distalen Bereich des zweiten Ballons und einem distalen Bereich des dritten Ballons des expandierbaren Elements (516) erstrecken, wobei der eine oder die mehreren Befestigungsarme (590) einen oder mehrere daran angeordnete Vorsprünge (594) aufweisen, wobei der eine oder die mehreren Vorsprünge (594) dazu ausgelegt sind, einen Abschnitt der linken Vorhofrohrwand in Eingriff zu nehmen.

4. Medizinische Vorrichtung (110) nach einem der Ansprüche 1-3, wobei das expandierbare Element (116) ein erstes Einwegventil aufweist, das dazu ausgelegt ist, zuzulassen, dass ein Aufblasmaterial zwischen der ersten Kammer (121) und der zweiten Kammer (123) fließt, wobei das erste Einwegventil dazu ausgelegt ist, sich zu öffnen, wenn die erste Kammer (121) auf einen ersten Schwellenaufblasdruck aufgeblasen wird.

5. Medizinische Vorrichtung (10) nach einem der Ansprüche 1-4, einen vierten Ballon (22), der eine vierte Aufblaskammer (29) in Fluidverbindung mit der ersten Aufblaskammer (21) definiert, und einen fünften Ballon (26), der eine fünfte Aufblaskammer (31) in Fluidverbindung mit der ersten Aufblaskammer (21) definiert, wobei der vierte und der fünfte Ballon (22, 26) in Umfangsrichtung um den ersten Ballon (18) beabstandet sind.

6. Medizinische Vorrichtung (310; 410; 510) nach einem der Ansprüche 1-5, ferner umfassend einen Vorsprung (394; 494; 594), der dazu ausgelegt ist, die medizinische Vorrichtung (310; 410; 510) an einer Zielgewebestelle des linken Vorhofrohrs (60) zu verankern.

7. Medizinische Vorrichtung (310; 410; 510) nach Anspruch 6, wobei der Vorsprung (394; 494; 594) dazu ausgelegt ist, zwischen einer ersten Position und einer zweiten ausgefahrenen Position zu wechseln, wobei sich der Vorsprung (394; 494; 594) in der zweiten ausgefahrenen Position radial von einer Außenfläche des ausdehnbaren Elements (316; 416; 516) weg erstreckt.

8. Medizinische Vorrichtung (210) nach einem der Ansprüche 1-7, ferner umfassend ein zweites Aufblasventilelement (272), das zwischen der ersten Aufblaskammer und der zweiten Aufblaskammer positioniert ist.

9. Medizinische Vorrichtung (210) nach Anspruch 8, wobei das expandierbare Element (16) dazu gestaltet ist, einen Aufblaskatheter (278) mit einer ersten Aufblasöffnung (280) und einer zweiten Aufblasöffnung (288) in Eingriff zu nehmen, und wobei sich die erste Aufblasöffnung (280) durch das zweite Aufblasventilelement (272) in die zweite Aufblaskammer erstreckt, und wobei die zweite Aufblasöffnung (288) innerhalb der ersten Aufblaskammer positioniert ist, wenn die erste Aufblasöffnung (280) innerhalb der zweiten Aufblaskammer positioniert ist.

10. Medizinische Vorrichtung (210) nach einem der Ansprüche 1-9, wobei das expandierbare Element (16) dazu ausgelegt ist, die zweite Kammer auf einen ersten Aufblasdruck aufzublasen, und wobei das expandierbare Element (16) dazu ausgelegt ist, die erste Kammer auf einen zweiten Aufblasdruck aufzublasen, nachdem die zweite Kammer auf den ersten Aufblasdruck aufgeblasen wurde, und wobei das zweite Aufblasventilelement (272) dazu ausgelegt ist, den ersten Aufblasdruck in der zweiten Kammer aufrechtzuerhalten, während die erste Aufblaskammer auf den zweiten Aufblasdruck aufgeblasen wird.

## Revendications

1. Dispositif médical (10) destiné à occlure un appendice auriculaire gauche (60) en bifurcation, comprenant :
un élément expansible (16) comprenant un premier ballonnet central (18) définissant une première chambre de gonflage (21), un deuxième ballonnet (20) définissant une deuxième chambre de gonflage (23) et un troisième ballonnet (24) définissant une troisième chambre de gonflage (27), la deuxième chambre de gonflage (23) étant adjacente à la première chambre de gonflage (21), la première chambre de gonflage (21) étant en communication fluidique avec la deuxième chambre de gonflage (23) et la troisième chambre de gonflage (27), les deuxième et troisième ballonnets (20, 24) étant espacés circonférentiellement autour du premier ballonnet (18), et l'élément expansible (16) étant conçu pour passer d'une première configuration non déployée à une deuxième configuration déployée ; et
un premier élément de valve de gonflage (32) s'étendant au moins partiellement dans la première chambre de gonflage (21),
l'élément expansible (16) étant configuré pour se déployer et fermer hermétiquement l'ouverture de l'appendice auriculaire gauche (60),
le deuxième ballonnet (20) et le troisième ballonnet (24) de l'élément expansible (16) étant allongés dans une plus grande mesure que le premier ballonnet (18) lorsque l'élément expansible (16) est dans la deuxième configuration déployée, et
le deuxième ballonnet (20) étant configuré pour se déployer afin de s'étendre dans un premier lobe (62) de l'appendice auriculaire gauche (60) et le troisième ballonnet (24) étant configuré pour se déployer afin de s'étendre dans un deuxième lobe (64) de l'appendice auriculaire gauche (60).

2. Dispositif médical (10) selon la revendication 1, l'élément expansible (16) étant configuré pour gonfler la première chambre (21) à une première pression de gonflage, et l'élément expansible (16) étant configuré pour gonfler la deuxième chambre (23) à une deuxième pression de gonflage après que la première chambre (21) a été gonflée à la première pression de gonflage.

3. Dispositif médical (10 ; 310) selon l'une quelconque des revendications 1 et 2, comprenant en outre un ou plusieurs bras de fixation (590) s'étendant depuis une région distale du deuxième ballonnet et une région distale du troisième ballonnet de l'élément expansible (516), le ou les bras de fixation (590) comprenant une ou plusieurs saillies (594) disposées sur ceux-ci, la ou les saillies (594) étant configurées pour venir en prise avec une partie de la paroi de l'appendice auriculaire gauche.

4. Dispositif médical (110) selon l'une quelconque des revendications 1 à 3, l'élément expansible (116) comprenant une première valve unidirectionnelle configurée pour permettre à un matériau de gonflage de s'écouler entre la première chambre (121) et la deuxième chambre (123), la première valve unidirectionnelle étant configurée pour s'ouvrir lorsque la première chambre (121) est gonflée à une première pression de gonflage de seuil.

5. Dispositif médical (10) selon l'une quelconque des revendications 1 à 4, un quatrième ballonnet (22) définissant une quatrième chambre de gonflage (29) en communication fluidique avec la première chambre de gonflage (21), et un cinquième ballonnet (26) définissant une cinquième chambre de gonflage (31) en communication fluidique avec la première chambre de gonflage (21), les quatrième et cinquième ballonnets (22, 26) étant espacés circonférentiellement autour du premier ballonnet (18).

6. Dispositif médical (310 ; 410 ; 510) selon l'une quelconque des revendications 1 à 5, comprenant en outre une saillie (394 ; 494 ; 594) configurée pour ancrer le dispositif médical (310 ; 410 ; 510) à un site tissulaire cible de l'appendice auriculaire gauche (60).

7. Dispositif médical (310 ; 410 ; 510) selon la revendication 6, la saillie (394 ; 494 ; 594) étant configurée pour se déplacer entre une première position et une deuxième position étendue, la saillie (394 ; 494 ; 594) s'étendant radialement à l'écart d'une surface extérieure de l'élément expansible (316 ; 416 ; 516) dans la deuxième position étendue.

8. Dispositif médical (210) selon l'une quelconque des revendications 1 à 7, comprenant en outre un deuxième élément de valve de gonflage (272) positionné entre la première chambre de gonflage et la deuxième chambre de gonflage.

9. Dispositif médical (210) selon la revendication 8, l'élément expansible (16) étant conçu pour venir en prise avec un cathéter de gonflage (278) ayant un premier orifice de gonflage (280) et un deuxième orifice de gonflage (288), et le premier orifice de gonflage (280) s'étendant dans la deuxième chambre de gonflage à travers le deuxième élément de valve de gonflage (272), et le deuxième orifice de gonflage (288) étant positionné à l'intérieur de la première chambre de gonflage lorsque le premier orifice de gonflage (280) est positionné à l'intérieur de la deuxième chambre de gonflage.

10. Dispositif médical (210) selon l'une quelconque des revendications 1 à 9, l'élément expansible (16) étant configuré pour gonfler la deuxième chambre à une première pression de gonflage, et l'élément expansible (16) étant configuré pour gonfler la première chambre à une deuxième pression de gonflage après que la deuxième chambre a été gonflée à la première pression de gonflage, et le deuxième élément de valve de gonflage (272) étant conçu pour maintenir la première pression de gonflage dans la deuxième chambre tandis que la première chambre de gonflage est gonflée à la de
